Europäisches Patentamt

European Patent Office

Office européen des brevets

(11) Publication number: 0 165 801

A2

# (12) EUROPEAN PATENT APPLICATION

(21) Application number: 85304334.7

(22) Date of filing: 17.06.85

(51) Int. Cl.⁴: **C 12 M 1/26**
**G 01 N 1/10**

(30) Priority: 19.06.84 JP 126137/84

(43) Date of publication of application:
27.12.85 Bulletin 85/52

(84) Designated Contracting States:
DE FR GB IT

(71) Applicant: KYOWA HAKKO KOGYO CO., LTD.
6-1, Ohte-Machi Itchome
Chiyoda-ku Tokyo(JP)

(72) Inventor: Hagino, Hiroshi
3-16-1-701, Kaminoge Setagaya-ku
Tokyo(JP)

(72) Inventor: Yotsuji, Akira
3-6-6, Asahi-machi
Machida-shi Tokyo(JP)

(74) Representative: Lambert, Hugh Richmond et al,
D. YOUNG & CO. 10 Staple Inn
London, WC1V 7RD(GB)

(54) Method and apparatus for aseptic sampling of fermentation liquor from a fermentation tank.

(57) According to the method and apparatus of the invention, a fermentation liquor is aseptically sampled by withdrawing a recycle stream of fermentation medium from the fermentation vessel (1), passing the recycle stream through a closed tubular loop (4) back into the fermentation vessel, the loop having at least one tubular section (3) constructed of a porous filter material permeable to the liquor, but not by the culture microorganism or other solids. Liquor which filters through the porous tubular section is collected as the sample. Internally the tubular section is kept free of deposits clogging the filter by the flow of liquid around the recycle loop.

FIG.1

EP 0 165 801 A2

METHOD AND

APPARATUS FOR ASEPTIC SAMPLING OF FERMENTATION

LIQUOR FROM FERMENTATION TANK

The present invention relates to a method and apparatus for aseptic sampling of a fermentation liquor from a fermentation tank.

For proper control of fermentation, it is necessary to accurately analyze the consumption of medium components and quantitative changes of microbial products contained in the fermentation liquor. Based on the consumption, short components are supplemented, and based on the increase or decrease of the fermentation products, fermentation operation is discontinued.

For example, for the normal fermentation, it is important to constantly and accurately analyze the consumption of sugars such as glucose, etc. added to a fermentation medium and supply thereto the necessary amount thereof, because a shortage of sugars or excessive addition of sugars gives an adverse effect on the growth of microorganisms or the accumulation of fermentation products. Thus, it is necessary to withdraw a fermentation liquor intermittently from a fermentation tank (the withdrawal may be hereinafter referred to as "sampling") for analysis.

To prevent the fermentation liquor from contamination with microorganisms, it is necessary to aseptically sample the fermentation liquor from the fermentation tank. Usually, the sampling of fermentation liquor is carried out by sterilizing a sampling nozzle and its adjacent part with steam or a disinfectant such as alcohol, etc. However, it is troublesome to conduct sterilization at every sampling, and sometimes it is preferable that a fermentation filtrate can be directly sampled.

On the other hand, studies have been made on methods using various filter materials for aseptic sampling. When a fermentation liquor is directly filtered in such methods, the pores of filter material are clogged with cells and insoluble components of the medium in the fermentation liquor, and thus the filtration capacity is rapidly lowered within a short time. That is, only a small amount of a sample can be obtained, and it is difficult to continuously use the filter without removing the clogging matters. To remove the solid components clogging the filter pores, it has been proposed to conduct back-washing with a solution or air from the outside to the inside. Even if the clogging matters could be removed by the back-washing and the sampling could be restarted, the clogging would take place again within a short time. To solve this problem, it has been proposed to provide sampling nozzles with filter materials at two places in a fermentation tank to sample a filtrate from one of the filters, while using the filtrate as back-washing water for the other filter through a communicating tube, and to switch the back-washing to the sampling after the washing and repeat the operations of sampling-washing alternately, thereby preventing the filter clogging [Isao Endo: Chemical Technical Report No. 2, page 63, compiled by Chemical Engineering Society, Study Team for New Utilization Technique of Microorganism Functions (1983)]. The problem of clogging will be partly solved in the said apparatus, but in the case of a fermentation liquor which has a particularly high viscosity, rapid clogging of the sampling filter will not be evaded, and thus it seems difficult to obtain filtrate enough for back-washing.

3

**0165801**

In one aspect of this invention there is provided a method of taking a sample of a culture liquor aseptically from a fermentation vessel containing a fermentation medium, which comprises intermittently or continuously withdrawing a recycle stream of fermentation medium from the fermentation vessel, passing that recycle stream through a closed tubular loop containing at least one tubular section constructed of a porous filter material that is permeable by the culture liquor, but impermeable to the culture microorganism and other solids present in the fermentation medium, returning said recycle stream via said loop to the fermentation vessel, and collecting as said sample, the liquor which filters through said porous tubular section of the loop. In a preferred technique the filtration of the liquor through the porous section is vacuum assisted, and the pores in the porous material are periodically cleaned by blowing a gas therethrough into the interior of the tube.

In a second aspect, there is provided a fermentation apparatus comprising a fermentation vessel and means for periodically taking a sample of the culture liquor aseptically from the fermentation vessel, wherein the sampling means comprise a conduit forming a closed loop with the fermentation vessel and comprising at least one tubular section constructed of a porous, filter material permeable by the culture liquor, but not by the culture microorganism or other solids contained in the fermentation medium, means for continuously or intermittently withdrawing a stream of fermentation medium from the fermentation vessel and recycling that stream through the conduit back into the fermentation vessel, and means for collecting the liquor sample as it filters through the porous tubular section or sections of the loop.

Preferably the collection means comprise a vessel hermetically sealed about said porous tubular section or sections of the loop, said vessel having a cock for recovering the sample from the vessel, and nozzle for connecting a vacuum or pressure line to the vessel for the evacuation or pressurisation thereof, respectively.

Using the method and apparatus of this invention a fermentation liquor can be aseptically sampled with a filter material over a long period of time without blockage of the filter.

The method and apparatus of this invention is further described with reference to the accompanying drawings in which:

Figure 1 shows the principle of the method and apparatus of the present invention in one embodiment; and

Figure 2 shows a second and improved embodiment of the invention.

Referring to the drawings a stream fermentation liquor is circulated in the direction of the arrows from a fermentation tank 1 with a pump 2 e.g. a peristaltic or roller pump through a filter section 3 consisting of a porous tube and through a tube 4 to the fermentation tank 1. At that time, the pressure inside the tube is the pressure in the fermentation tank plus the discharge pressure of the pump, and the culture liquor is filtered from the inside of the filter to the outside by a pressure difference between the said pressure and the atmospheric pressure, and taken out, whereby a clear liquor free from solid matters and cells can be obtained. This apparatus is characterized in that the fermentation liquor from the fermentation tank can be passed through the filter section, and circulated and returned to the fermentation tank at any time, when required, whereby the fermentation liquor is filtered through the filter material and, at the same time, the inside surface of the filter is washed with the liquor stream to prevent the filter pores from clogging. It is necessary that the filter section is in a tubular form so that the liquor can flow therethrough without any stagnation and the inside surface of the filter can be washed continually. The shape of the filter tube in the longitudinal direction can be straight or curved. A plurality of filter tubes can be connected in series or in parallel. The filter section and nozzle 5 of the fermentation tank are generally connected with each other through tubes, usually flexible tubes, by means of flanges, couplers, etc. or by directly inserting the tubes into the nozzle. If they can be directly connected with each other without any tube, it is not necessary to use a tube.

Filter material can include inorganic materials such as glass, ceramics, silicone, etc.; organopolymeric materials such as polyvinyl alcohol, Teflon, polypropylene, nylon, cellulose-based resin, etc.; and metallic materials such as porous stainless steel, etc., and so on. Further, any other material can be used so long as it is a uniform porous material and can be sterilized with steam or a chemical.

0165801

Pore diameter of the filter material is usually 0.01 to 10 μ, preferably 0.1 to 10 μ in view of the condition that the cultured microorganisms do not leak out or, to the contrary, contaminating microorganisms do not pass therethrough from the outside, and in view of prevention of pore clogging. Material for tube 4 may be silicone, Teflon, polyvinyl chloride, synthetic rubber, or any organopolymeric material that can withstand autoclave sterilization or chemical sterilization.

One example of the present invention is described in detail below.

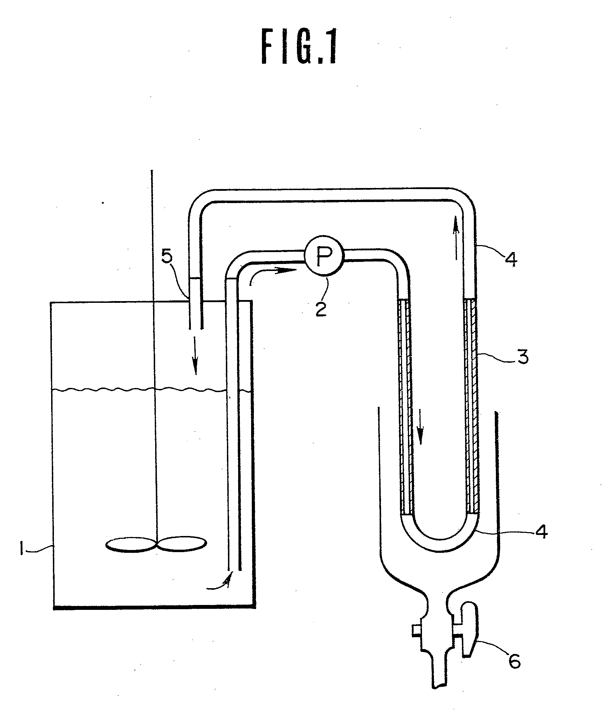

The present inventors connected a ceramic tube filter material having a pore diameter of 1.0 to 1.5 μ to a silicone tube, sterilized the ceramic tube and the silicone tube in an autoclave, and then connected them to a fermentation tank as shown in Fig. 1. The silicone tube was connected to a tube pump and a fermentation liquor was circulated through the tube and returned to the fermentation tank by working the pump.

This apparatus was used for sampling fermentation liquors obtained by using Saccharomyces cerevisiae, Bacillus subtilis, Escherichia coli and Corynebacterium glutamicum, and any of the fermentation filtrates obtained by filtration through the filter section was a clear liquor entirely free from the cells. The apparatus was operated for a long time from the start to the end of culturing during which sampling was conducted at regular intervals of time. In the case of a fermentation liquor at the initial stage of culturing having low viscosity and a low cell content, it was possible to conduct sampling at a constant sampling rate for a long time. On the other hand, in the case of a fermentation liquor at the later stage of culturing having high viscosity and high cell content, filter pores were gradually clogged by sampling for a long time, the filtration efficiency was lowered, and the time required for sampling a predetermined amount of filtrate became longer. However, when the fermentation time was not very long, as in the case of batch culturing, it was 0165801 not impossible to conduct sampling even at the later stage of culturing. When the sampling was to be conducted for a long time, as in the case of continuous fermentation, it was necessary to properly exchange the sampling apparatus. In this case, the used sampling apparatus was made reutilizable by washing the filter section with water or acid to remove the clogging matters therefrom, and then sterilizing the apparatus.

Sampling could be similarly conducted by the above procedures employing a porous glass tube filter material having a pore diameter of 0.1 μ.

If the apparatus can be used for a long time without any exchange of the filter section, it will be more advantageous for sampling of culture liquor for a long time from the standpoint of labor saving and keeping of aseptic conditions. As a result of further extensive studies of these aspects, the present inventors have completed an improved sampling apparatus which withstands prolonged continuous use. The improved apparatus is characterized in that the filter section of the said apparatus is encased in a vessel in a tightly sealed state, and that the pressure inside the vessel can be increased by introducing a gas (usually air) from the outside into the vessel or reduced by letting a gas out of the vessel.

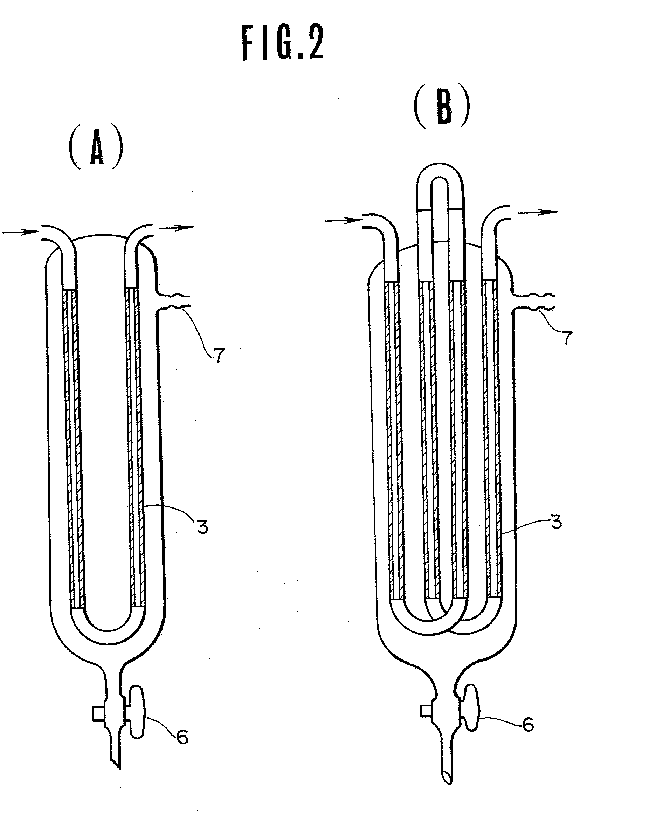

An example of the improved apparatus according to the present invention is described below, referring to Fig. 2.

Members other than the filter section in Fig. 2 (A) are the same as used in Fig. 1. In the present apparatus, the filter section is sealed in a glass cylinder, and the filtrate passed through the filter material can be withdrawn to the outside by opening a cock 6. Usually, the filtration of a fermentation liquor can be sufficiently carried out at atmospheric pressure in the glass cylinder, but, if necessary, the filtration may be carried out under reduced pressure by decompressing the inside of the glass cylinder with the pump through a nozzle 7. After the sampling, the pressure in the glass cylinder can be raised by supplying air with the pump through the nozzle 7 to apply a pressure to the filter surface from the outside to the inside, whereby back-washing of the filter and removal of the clogging matters are effected. As for the degree of pressure application, it is enough to apply a pressure of at least 0.2 to 0.3 $kg/cm^2$ plus the pressure inside the fermentation tank. By repetition of the sampling at atmospheric pressure or reduced pressure and the successive back-washing under raised pressure using the present apparatus, even a fermentation liquor containing a high cell content or a highly viscous fermentation liquor can be filtered efficiently, and a filtrate can be withdrawn. Thus, the present apparatus can be used as a sampling apparatus for prolonged continuous fermentation without any exchange of filter section. Sampling takes a short time as the filtration surface area of the filter section is broader. Thus, 2 - 4, or more filter tubes may be connected to one another, if necessary, as shown in Fig. 2 (B). The cylinder in which the filter section is tightly sealed can be made of any material, so long as it can withstand the applied pressure (positive pressure or negative pressure) and can also be sterilized with steam or a chemical.

The circulation line for a fermentation liquor from a fermentation tank and the entire sampling tube including the filter material sealing section are sterilized in advance, an aseptic filter is provied at the nozzle 7, and sterilized air is introduced or withdrawn to or from the filter material sealing section, whereby the entire sampling apparatus is kept under sterile conditions and the withdrawn sample is free from contamination with microorganisms. In this state, the present sampling apparatus can withstand prolonged use. It is, of course, possible to automate the entire sampling apparatus of the present invention by automatically switching the rotating

0165801

direction of the pump connected to the nozzle 7 with a timer to automate the operation of raising or reducing the pressure and by making the valve of cock 6 automatic.

As described in detail above, the present invention can provide an apparatus for aseptic stable sampling for a long time from the start to the end of culturing.

## CLAIMS

1.    A method of taking a sample of a culture liquor aseptically from a fermentation vessel containing a fermentation medium, characterised by intermittently or continuously withdrawing a recycle stream of fermentation medium from the fermentation vessel (1), passing that recycle stream through a closed tubular loop (4) containing at least one tubular section (3) constructed of a porous filter material that is permeable by the culture liquor, but impermeable to the culture microorganism and other solids present in the fermentation medium, returning said recycle stream via said loop to the fermentation vessel (1), and collecting as said sample, the liquor which filters through said porous tubular section (3) of the loop (4).

2.    A method according to claim 1, characterised in that the filtration of the culture liquor through the porous filter material constituting said section (3) of the loop (4) is vacuum assisted.

3.    A method according to claim 1 or 2, characterised in that the pores of the porous filter material constituting said tubular section (3) are periodically cleaned by applying a positive gas pressure externally of the tubular section thereby to blow gas thereinto through said pores.

4.    Fermentation apparatus comprising a fermentation vessel (1) and means (2,3,4,5,6) for periodically taking a sample of the culture liquor aseptically from the fermentation vessel, characterised in that the sampling means comprise a conduit (4) forming a closed loop with the fermentation vessel (1) and comprising at least one tubular section (3) constructed of a porous, filter material permeable by the culture liquor, but not by the culture microorganism or other solids contained in the fermentation medium, means (2) for continuously or intermittently withdrawing a stream of fermentation medium from the fermentation vessel and recycling that stream through the conduit (4) back into the fermentation vessel (1), and means for collecting the liquor sample as it filters through the porous tubular section or sections (3) of the loop (4).

5.    Apparatus according to claim 4, characterised in that the collection means comprise a vessel hermetically sealed about said porous tubular section or sections (3) of the loop (4), said vessel having a cock (6) for recovering the sample from the vessel, and nozzle (7) for connecting a vacuum or pressure line to the vessel for the evacuation or pressurisation thereof respectively.

6.    Apparatus according to claim 4 or 5, characterised in that the means (2) for withdrawal and recycle of the fermentation medium through said conduit (4) is a peristaltic pump.

# FIG.1

FIG.2

(A)

(B)